# EUROPEAN PATENT APPLICATION

(11) **EP 1 553 395 A1**
(43) Date of publication of application: **13.07.2005**
(21) Application number: 03756595.9
(22) Date of filing: 18.09.2003
(51) Int. Cl.: G01N 1/10

(54) **BLOOD ANALYZER AND METHOD OF SEPARATING PLASMA**

(30) Priority: 20.09.2002 JP 2002275853
(71) Applicant: Japan Science and Technology Agency, Kawaguchi-shi, Saitama 332-0012 (JP)
(72) Inventor: OGAWA, Hiroki, 303 Yokohama Island Garden,, Yokohama-shi, Kanagawa 230-0031 (JP); HORIIKE, Yasuhiro, Nishitokyo-shi, Tokyo 202-0021 (JP)
(74) Representative: Grünecker, Kinkeldey, Stockmair & Schwanhäusser Anwaltssozietät
(86) International application number: PCT/JP2003/011909
(87) International publication number: WO 2004/027391

(57) **Abstract**

In a blood analyzer for separating plasma in a flow channel by centrifugal operation, it is intended to effectively utilize a whole blood sample supplied into the flow channel, shorten the flow channel and reduce the apparatus size. It is also intended to reduce the amount of the blood to be collected, thereby reducing the burden on a subject. A blood cell reservoir wherein blood cells are precipitated is provided in a flow channel of a blood analyzer along the centrifugal direction upon centrifugation. Then blood cells are cumulated in the reservoir by centrifugation so that the plasma fraction is allowed to continuously exist in both of the upstream and downstream sides of the U-shaped flow channel without being divided by the blood cell fraction. Thus, the plasma in a required amount can be fed into the analysis means using a smaller amount of the whole blood. Therefore, the whole blood can be utilized more efficiently, which is suitable for the shortening of the flow channel and the reduction of the device size. The plasma, which is not divided by the blood cell fraction, can be transferred due to a lower negative suction pressure. Thus, the pump capacity required in drawing the plasma can be reduced, which contributes to the size reduction and cost down of peripheral devices.

## Description

### Technical Field

The present invention relates to a chip-shaped blood analysis apparatus constituted by micro trench channels prepared in insulating substrates such as a quartz plate and a polymer resin plate. Particularly, the present invention relates to a plasma separation method for utilizing plasma fraction efficiently, when a small amount (several µL or less) of blood is introduced in a trench channels of the chip to perform centrifugal separation, the blood is separated into blood cell fraction and plasma fraction, and thereafter various chemical material concentrations in the plasma are measured, and a trench channel structure of the chip-shaped blood analysis apparatus.

### Background Art

In a conventional medical check-up or diagnosis of a disease state, several cc, a large amount of blood has heretofore been sampled from a patient, and analysis has been carried out by a large-scaled automatic blood analysis apparatus. Usually, this automatic analysis apparatus is large in size, and therefore is installed in a medical institution such as a hospital. Further, the apparatus is operated only by a person who has specialty qualification.

However, in recent years, there is increased a demand to develop a new device enable to grasp health condition of a patient quickly and put such device to practical use. To the device, a fine working technique for use in preparing an extremely advanced semiconductor device is applied, analysis devices such as various sensors are arranged on a chip having a size of a several mm to several cm square at most, and body fluids of a person being tested is applied to the device. By development of such inexpensive device, daily health cares of aged people could be managed at home in a coming aging society, and accordingly a health insurance benefit tracing a course to an increase would be compressed. Such device may realize quick diagnosis of presence of an infectious disease (hepatitis, acquired immune deficiency syndrome, etc.) of the person being tested and proper action thereafter in the field of the emergency medical care. Thus, various social effects could be expected, and therefore the device is in a technical field which has gotten a lot of attention. In this situation, in lieu of the conventional automatic analysis apparatus, there have been developed a small-sized simple blood analysis method and blood analysis apparatus for personally performing blood analysis at home (e.g., Unexamined Japanese Patent Publication (KOKAI) JP 2001-258868 A).

FIG. 1 shows one example of a blood analysis apparatus formed as a micro module described in JP 2001-258868 A. Reference numeral 101 denotes a lower substrate of the blood analysis apparatus, and a micro trench channel (microcapillary) 102 is formed on the lower substrate by etching. An upper substrate (not shown) having the same size substantially is laminated onto the lower substrate 101 to seal the microcapillary 102 from the outside.

In the flow channel 102, blood sampling means 103, plasma separating means 104, analysis means 105, and moving means 106 are successively disposed from a most upstream portion toward a most downstream portion. A hollow blood collecting needle 103a is attached to the blood sampling means 103 which is provided most upstream end of the flow channel. The human body is stung with the needle 103a so that the needle constitutes an intake port of the blood into the substrate. The separating means 104 is formed by bending the flow channel 102 midway, and is constituted of, for example, U-shaped microcapillary. After introducing the sampled blood into this U-shaped microcapillary, acceleration is applied to the substrate in a certain direction by a centrifuge, blood cell components are precipitated in a U-shaped lowermost portion, and plasma is separated as supernatant. The analysis means 105 includes sensors for measuring a pH value, and concentration of each of oxygen, carbon dioxide, sodium, potassium, calcium, glucose, lactic acid and the like in the blood.

The moving means 106 positioned in the most downstream portion of the flow channel moves the blood within the microcapillary by an electro osmotic flow. The moving means 106 is constituted of electrodes 107, 108 and a flow channel portion 109 connecting both electrodes. A buffer solution with which the flow channel is filled previously is moved into the downstream side of the flow channel by the electro-osmosis flow generated by application of a voltage between the electrodes. And the blood is taken into the substrate from the blood sampling means 103 disposed at the front end of the flow channel 102 by a generated suction force. The plasma obtained by centrifugal separation is fed into the analysis means 105.

Reference numeral 110 denotes output means for taking information out of the analysis means, and comprises electrodes and the like, and 111 is control means for controlling the above-described sampling means, plasma separating means, analysis means, moving means, and output means, as needed.

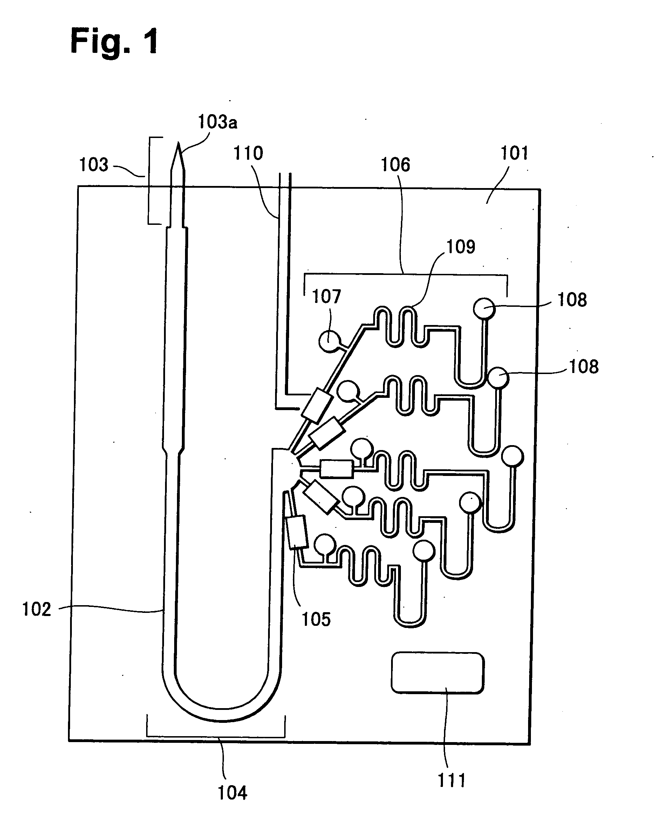

The blood collected by the sampling means 103 is separated into plasma and blood cell components by the separating means 104, and the plasma is transferred into the analysis means 105. Then, the pH value in the plasma, and the respective concentrations of oxygen, carbon dioxide, sodium, potassium, calcium, glucose, lactic acid and the like in the plasma are measured. The movement of the blood between the respective means is performed by the moving means 106 using phenomena such as electrophoresis and electro-osmosis. In FIG. 1, a downstream region of the flow channel 102 is branched into five, and each branch is provided with the analysis means 105 and moving means 106.

A glassy material such as quartz has been often used as a material for the substrate of the blood analysis apparatus, but a resin material has been regarded as more suitable for mass-producing the apparatuses at a reduced cost, and used.

In the prior blood analysis apparatus as shown in FIG. 1, the blood is collected by the sampling means 103, and separated into the plasma and blood cell components by the separating means 104, the separated plasma are fed into the analysis means, and various components in the plasma are analyzed. However, when the blood is separated into the plasma and blood cell components by the centrifugation in the separating means, the flow channel becomes plugged with the blood cell fraction precipitated in the lowermost portion of the U-shaped flow channel, and the plasma isolated as the supernatant is divided into an upstream side portion 102a and a downstream side portion 102b of the U-shaped flow channel. Therefore, there has been a problem that the only plasma on the downstream side can be fed into the analysis means, and the plasma on the upstream side cannot be utilized.

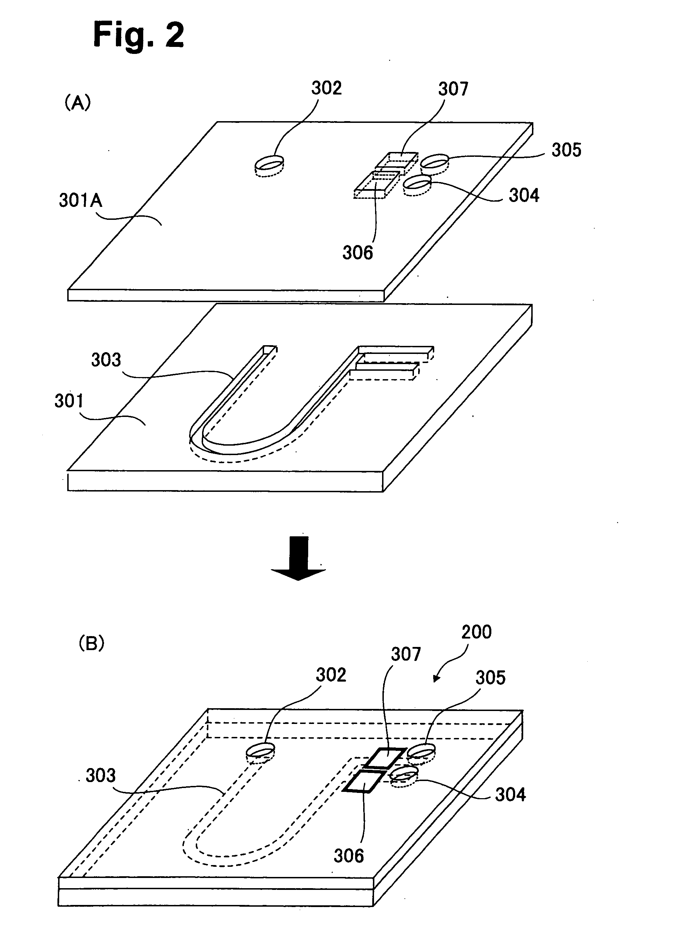

This state will be described briefly with reference to FIG. 2. FIG. 2 shows a state of preparation of the prior blood analysis apparatus. First, as shown in the part (A) of the figure, two substrates, for example, a lower substrate 301 and an upper substrate 301A formed of materials such as resin are provided, and a trench channel 303 having a width of about 100 microns and a depth of about 100 microns is formed by a method such as molding process. As shown in the figure, a part of the trench channel 303 includes a U-shaped flow channel. The upper substrate 301A comprises an input-side through hole 302 for introducing blood to be analyzed therein, output-side through holes 304, 305 for connecting an external pump to take the blood into the flow channel, and analysis means 306, 307 for detecting different components in the plasma. Both substrates 301, 301A2 are bonded to each other by pressure welding, adhesive, adhesive tape and the like to prepare a blood analysis apparatus 200 (FIG. 2(B)).

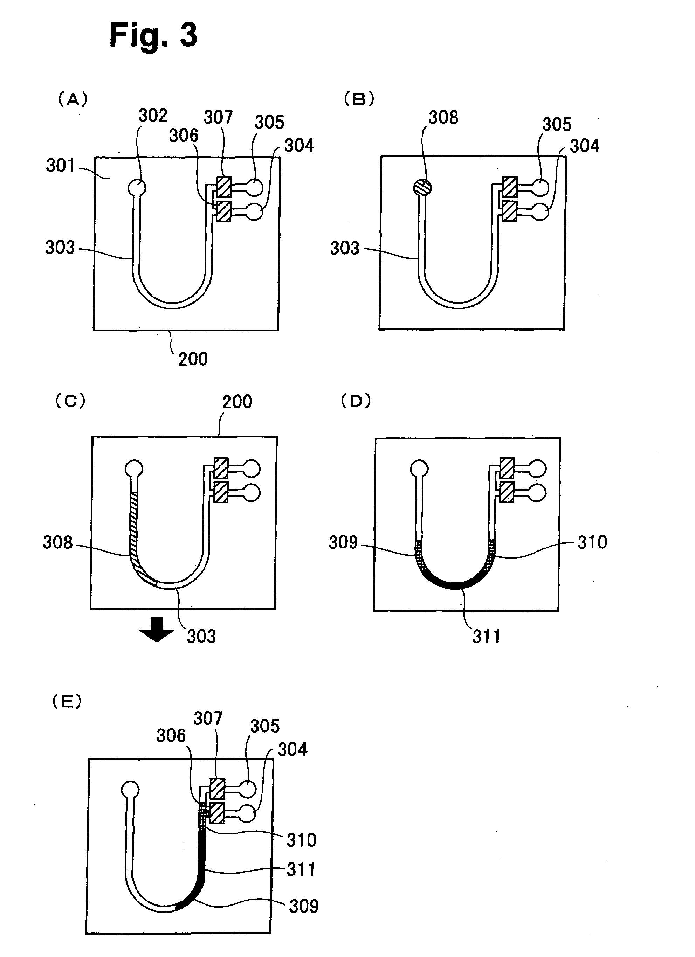

FIG. 3 includes schematic plan views of the thus prepared blood analysis apparatus, and also shows a state in which human whole blood is introduced into the flow channel of the blood analysis apparatus and is subjected to centrifugation. About 1 µL of blood is dripped into the input-side through hole (blood inlet port) 302 of the blood analysis apparatus of FIG. 3(A) (FIG. 3(B)), and whole blood 308 is introduced into the flow channel 303 by suction pump via the output-side through holes (outlet ports) 304, 305 (FIG. 3(C)). Next, the blood analysis apparatus 200 is rotated to perform centrifugal separation in such a manner that force is exerted in a U-shaped flow channel direction (arrow direction in FIG. 3(C)). As shown in FIG. 3(D), the whole blood is separated into plasma fractions 309, 310 on opposite sides of the U-shaped flow channel 303, and a blood cell fraction 311 in the lower portion of the U-shaped flow channel. Thereafter, the output-side through holes 304, 305 are connected to the pump and the like, the downstream-side plasma fraction 310 is fed into the analysis means 306, 307 (FIG. 3(E)), and the respective detections or concentration measurements of the suspected chemical materials are performed there.

However, this U-shaped flow channel is plugged with the blood cell fraction 311, and therefore the upstream-side plasma fraction 309 shown in FIG. 3(D) cannot be fed into the analysis means for use. This means that a blood-collecting amount necessary for clinical inspection is unnecessarily increased. If the plasma fraction on the upstream side can be also fed into the analysis means, the blood amount necessary for the clinical inspection can be simply decreased to about half. This will reduce a total length of the flow channel, and further miniaturization of the blood analysis apparatus will be also possible.

Moreover, in the prior blood analysis apparatus of FIGS. 1 to 3, when the downstream-side plasma fraction 310 is fed into the analysis means 306, 307 after the centrifugation of the whole blood, the blood cell fraction 311 and the upstream-side plasma fraction 309 have to be simultaneously moved, as seen in FIG. 3(E). Since the blood cell fraction 311 sticks to the inner wall of the flow channel 303 at this process, there has been a problem that a suction force or pump force larger than that for sucking the whole blood into the flow channel before centrifugation is required for moving these fractions by the pump.

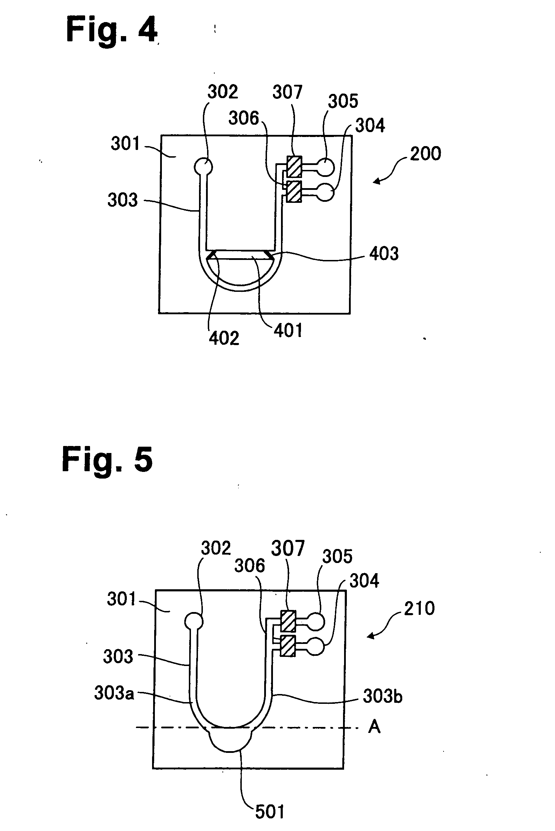

When a new bypass channel 401 as means for solving the above-described problem is provided between the upstream side and the downstream side of the U-shaped flow channel as shown in FIG. 4, the upstream-side plasma can be fed into a downstream side after the centrifugation. However, for this purpose, this bypass channel needs to be closed until the completion of the centrifugal separation, and new valves 402, 403 have to be installed in an inlet/outlet of the bypass channel 401. Since these valves also have to be controlled, the apparatus becomes complicate and large in size, and therefore, such arrangement is not realistic.

In such a circumstance, the present inventors have focused their attentions on the phenomenon such that the blood cell components after the centrifugation stick to the inner wall of the flow channel. Conversely, utilizing this phenomenon, attempts have been made to overcome the problem of the prior apparatus only by slight improvement of a flow channel design. That is, a portion of the flow channel in which the blood cell components are accumulated during the separation of the blood cells and the plasma by the centrifugation is formed to be thicker than the other portion of the flow channel. The blood cell components are accumulated in the thicker reservoir portion, and the plasma on the upstream and downstream sides are continuously connected to each other by the plasma via an upper portion of the reservoir portion, in which any blood cell is not accumulated. Thereafter, when the plasma is drawn into the analysis means by the pump, all the separated plasma can be fed into the analysis means without very large pump force, since the plasma on the upstream side is continuously connected to the plasma on the downstream side.

That is, a first object of the present invention is to provide a blood analysis apparatus which is an automatic analysis apparatus to separate the plasma by a centrifugal operation in a flow channel and which intends to efficiently utilize a whole blood sample supplied into the flow channel and which is suitable for the shortening of the flow channel and the reduction of the apparatus size and which can reduce the amount of the blood to be collected, thereby reducing the burden on a subject.

Moreover, another object of the present invention is to provide a plasma separation method capable of efficiently utilizing a whole blood sample supplied into a flow channel, when using an automatic analysis apparatus which separates plasma in the flow channel by a centrifugal operation.

### Disclosure of the Invention

According to the present invention, a first object is achieved by a blood analysis apparatus comprising: a flow channel which connects between a blood inlet port and an outlet port; and plasma separating means disposed midway in the flow channel,
wherein said flow channel has an upstream portion of the flow channel elongated along a centrifugal force pressurizing direction and a downstream portion elongated in a direction opposite to the a centrifugal force pressurizing reverse direction;
wherein said plasma separating means is positioned between the upstream and downstream portions of the flow channel and includes a blood cell fraction container which is located in the centrifugal force pressurizing direction side, and in which a blood cell fraction is precipitated and received; and
wherein the upstream and downstream portions of said flow channel are brought into contact with the blood cell fraction container, and are constituted to communicate with each other in an upper space of the blood cell fraction container.

For example, when a part of the flow channel is formed into a U-shaped flow channel, a lowermost portion (portion to which a centrifugal force G is applied) of the U-shaped flow channel may serve as the blood cell fraction container. This blood cell fraction container may be any space that protrudes downwards (centrifugal G loading direction) from the lowermost portion of the U-shaped flow channel. When the volume of the blood cell fraction container positioned in a centrifugal force pressurizing direction and positioned beneath an upper inner wall of the bottom portion of the U-shaped flow channel is larger than the amount of the blood cell fraction in the blood supplied into the flow channel, supernatant plasma of the upstream side can be connected to that of the downstream side via the upper space of the blood cell fraction container.

Analysis means for analyzing components in the plasma may be disposed between the plasma separating means and a outlet port. When a blood collecting needle is attachable to the blood inlet port, whole blood sampled from the blood collecting needle can be directly fed into the flow channel.

A second object of the present invention is achieved by a plasma separation method comprising the following steps of:
(1) providing a chip-shaped blood analysis apparatus comprising:
   a flow channel for connecting a blood inlet port to an outlet port, the flow channel having an upstream portion elongated along a centrifugal force pressurizing direction from the blood inlet port and a downstream portion elongated in a direction-opposite to the centrifugal force pressurizing direction; and
   plasma separating means located between the upstream and downstream portions of the flow channel and including a blood cell fraction container which is located in a centrifugal force pressurizing direction side and in which the blood cell fraction is precipitated and received, the upstream and downstream portions of the flow channel being brought into contact with the blood cell fraction container and constituted to communicate with each other in an upper space of the blood cell fraction container;
(2) introducing a whole blood sample into the flow channel from the blood inlet port; and
(3) centrifuging the blood analysis apparatus in such a manner that the blood cell fraction container is disposed in the centrifugal force pressurizing direction so as to precipitate blood cell components in a blood sample in said blood cell fraction container, so that the plasma separated as centrifugal supernatant is allowed to continuously exist in both of the upstream and downstream portions of the flow channel while the plasma contacts with blood cell fraction in the blood cell fraction container.

When analysis means for analyzing the components in the plasma is provided between the plasma separating means and the outlet port of the blood analysis apparatus, the plasma continuously existing in both of the upstream and downstream sides of the flow channel can be fed into the analysis means.

### Brief Description of the Drawings

FIG. 1 is an explanatory view of a prior chip-shaped blood analysis apparatus;
FIG. 2 is a view for explaining steps of preparing the prior chip-shaped blood analysis apparatus;
FIG. 3 is a diagram showing a state in which whole blood is supplied into the blood analysis apparatus of FIG. 2, and separated into plasma and blood cell fractions by centrifugation;
FIG. 4 is a diagram showing an alternative example of a constitution of a prior blood analysis apparatus;
FIG. 5 is a schematic diagram of an embodiment of a blood analysis apparatus according to the present invention;
FIG. 6 includes explanatory views, each showing steps of introducing blood into the blood analysis apparatus of FIG. 5, separating the blood into plasma and blood cell fractions, and thereafter feeding the plasma fraction into analysis means;
FIG. 7 is an explanatory view of a state in which the blood analysis apparatus of the present invention is mounted in a centrifugal separation apparatus to perform centrifugation;
FIG. 8 is an explanatory view of one example of a constitution of the blood analysis apparatus of FIG. 5 according to the present invention;
FIG. 9 shows a schematic diagram of the blood analysis apparatus of the present invention for use in a second example, and an explanatory view of a state after introducing the blood into the apparatus to perform the centrifugal separation;
FIG. 10 shows a schematic diagram of the blood analysis apparatus of the present invention for use in a third example, and an explanatory view of a state after introducing the blood into the apparatus to perform the centrifugal separation;
FIG. 11 shows a schematic diagram of the blood analysis apparatus of the present invention for use in a fourth example, and an explanatory view of a state after introducing the blood into the apparatus to perform the centrifugal separation; and
FIG. 12 shows a schematic diagram of the blood analysis apparatus of the present invention for use in a fifth example, and an explanatory view of a state after separating the plasma from the blood by a plasma separation method of the present invention.

### Best Mode for Carrying out the Invention

### Embodiment

FIG. 5 shows an embodiment of a blood analysis apparatus having a flow channel according to the present invention. The present blood analysis apparatus 210 basically has substantially the same constitution as that of an apparatus 200 shown in FIG. 3, but is different in that the lowermost portion of the U-shaped flow channel 303 to which acceleration of gravity is most applied by a centrifugation is constricted and broadened to form a blood cell reservoir (blood cell fraction container) 501. Since the same constituting portions are denoted with the same reference numerals, description will not be repeated.

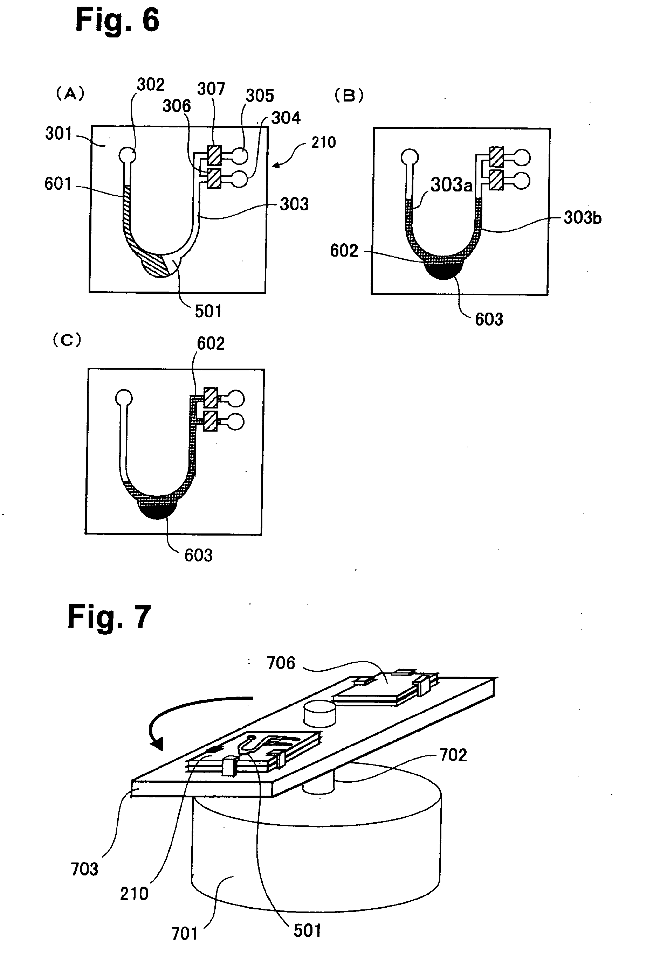

A state of an operation of the blood analysis apparatus will be described hereinafter. After introducing whole blood into the blood analysis apparatus as shown in FIG. 6(A), the blood analysis apparatus 210 is set onto a centrifugal separation apparatus shown in FIG. 7, and then the whole blood is subjected to centrifugation. At this time, the blood analysis apparatus 210 is disposed so that the blood cell reservoir 501 of the blood analysis apparatus is positioned in the radial direction of centrifugation, and a blood cell fraction is precipitated in the blood cell reservoir 501 by centrifugation. In additions, reference numeral 701 denotes a motor, 702 denotes a motor shaft, 703 denotes a chip support plate, and 706 denotes a balancer chip.

FIG. 6(B) shows a state of an inner space of the flow channel of the blood analysis apparatus 210 after a centrifugal operation. A blood cell 603 is centrifugally precipitated to be fractionated in a lower part of the blood cell reservoir 501, and plasma 602 is separated as a supernatant in the flow channel 303 above the blood cell fraction. At this time, unlike the conventional blood analysis apparatus (see FIG. 3), it is important that the plasma fraction should continuously exist in both of the upstream portion 303a and the downstream portion 303b in a U-shaped flow channel without being divided by the blood cell fraction.

For this reason, as shown in FIG. 5, a capacity of the blood cell reservoir (blood cell component container) 501 positioned under a line A, which is an upper end of an inner wall of the lowermost portion of the flow channel 303, is set to be larger than the amount of the blood cell component in the whole blood supplied into the flow channel 303. Since a human hematocrit value is 50% or less in general, the capacity of the blood cell reservoir (blood cell component container) 501 is preferably set to 1/2 or more of the amount of a sampled blood. The capacity of the blood cell reservoir can be determined in consideration of a length and sectional area of the U-shaped flow channel. In a case that the hematocrit value is high and the blood cell is expected to be fractionated beyond an upper end A of the blood cell reservoir, the separation of the blood cell and plasma can be performed by reducing the sampled blood amount.

After the centrifugation, an external suction pump is connected to suction ports (outlet ports) 304, 305 disposed to the most downstream end of the flow channel 303, and the plasma fraction 602 is drawn into analysis means 306, 307. Since the plasma fractions 602 of the downstream portion 303b and the upstream portion 303a of the U-shaped flow channel are not interrupted by the blood cell fraction 603, all the separated plasma fractions can be fed into the analysis means 306, 307 (FIG. 6(C)).

The blood cell fraction has been fixed to the inner wall of the bottom of the blood cell reservoir 501 by the gravitation due to centrifugation. Therefore, when the plasma fraction is suctioned to be transferred into the analysis means, the blood cell fraction does not move. The transfer of the plasma by suction can be performed only by movement of the plasma fraction which is a fluid element, and a large pump force is not required unlike a conventional case in which the blood cell fraction had to be moved together.

The blood cell reservoir in which the blood cell is precipitated in the radial direction of the centrifugation is provided in the flow channel of the blood analysis apparatus in this manner, and the blood cell is accumulated into the blood cell reservoir by the centrifugation. Moreover, the plasma fractions in both of the upstream and downstream sides of the U-shaped flow channel can continuously exist without being divided by the blood cell fraction. Then, all the separated plasma fractions can be taken into the analysis means, and a pump force required for drawing in the fractions may be small. A dimension of the blood cell reservoir can be determined from the dimension of the U-shaped flow channel and the amount of the whole blood to be subjected to centrifugation, with considering that the amount of the blood cell component shares 40 to 50% (volume) of the whole blood.

In this embodiment, the introduction of the blood and the suction transfer of the plasma fraction are performed by using the external pump, but moving means utilizing electro-osmosis flow may be disposed between the analysis means and the suction port (outlet port) like as in the conventional apparatus of FIG. 1. In this case, the outlet ports 304, 305 also serve as drain outlet ports for discharging buffer solutions with which the flow channel is filled.

### First Example

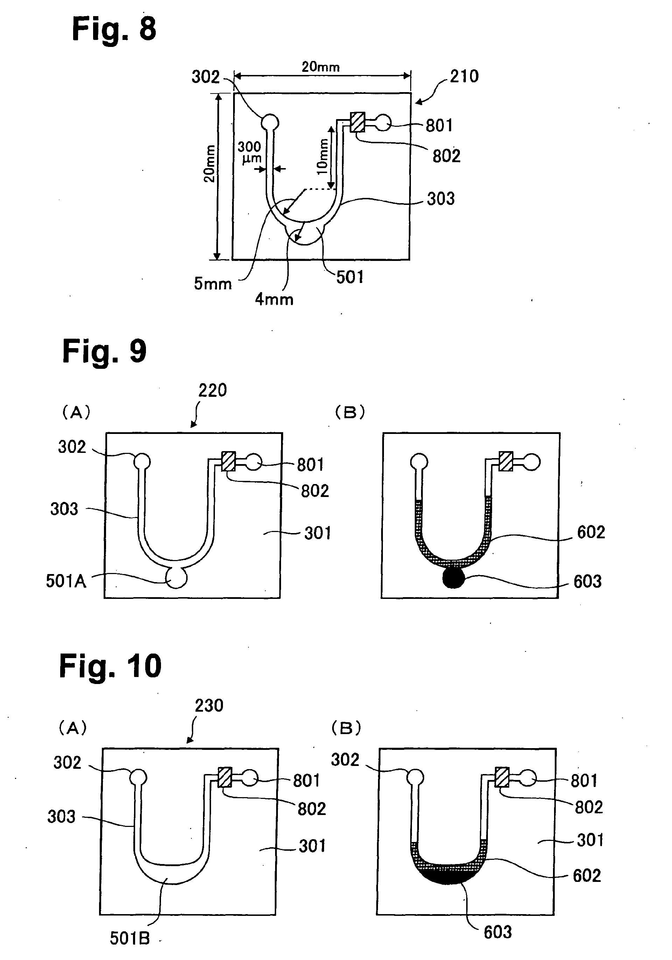

A blood analysis apparatus shown in FIG. 5 was prepared, a blood sample was introduced into the blood analysis apparatus to perform centrifugation, and thereafter the plasma fraction was sucked. Additionally, one analysis means was disposed in the blood analysis apparatus for simplicity (see FIG. 8). In the blood analysis apparatus, two polyethylene terephthalate (PET) substrates having a thickness of 0.5 mm were provided, and the flow channel 303 was formed in one substrate by molding. In the other substrate, a blood inlet port 302 and a blood draw-in port (outlet port) 801 were formed. And an electrode formed of carbon paste which was coated with a hydrogen ion sensitive film was prepared and disposed as analysis means 802. FIG. 8 schematically shows dimensions of a blood analysis apparatus 210 and the flow channel 303 formed on the apparatus. A depth of all the flow channel was 100 µm.

1 µL of human whole blood was introduced into the blood analysis apparatus 210 through the blood inlet port 302. A suction force of an electromagnetic pump attached to the outlet port 801 was used in introducing the blood. At this time, a suction negative pressure was -7 kPa relative to the atmospheric pressure. After introducing the blood, the blood analysis apparatus was centrifuged by a centrifugal apparatus shown in FIG. 7 (10,000 rpm, 2,250G for 1 min). In the flow channel of the blood analysis apparatus after centrifugation, as shown in FIG. 6(B), a blood cell fraction 603 was precipitated in the bottom of the blood cell reservoir 501. Plasma was isolated in such a manner as to continuously exist in the upper part of the blood cell reservoir and upstream and downstream sides of the U-shaped flow channel without being insulated from each other by the blood cell components. Thereafter, suction ports 304, 305 disposed on the downstream side of the analysis means were connected to the electromagnetic pump, and the plasma were transferred into the analysis means. At this time, the blood cell fraction did not move, and the only plasma fraction moved. Here, a hydrogen ion concentration in the plasma was analyzed to obtain the value of pH 7.4, which was substantially equal to a plasma pH value of a healthy person. With suction pressure of -7 kPa than the atmospheric pressure which was equal to the negative suction pressure for drawing in the whole blood, the plasma could be transferred without any problem.

Moreover, the blood analysis apparatus of FIG. 8 was modified, and a similar test was conducted even in a case where a hollow blood-collecting needle having an outer diameter of 100 µm and inner diameter of 50 µm was attached to the blood inlet port 302. First, a human forearm part was stung with the blood-collecting needle of the blood analysis apparatus, and about 1 µL of blood was collected and supplied into the blood analysis apparatus. Thereafter, the blood was separated into plasma and blood cell fractions by centrifugation in the same manner as in the above-described procedure, and the only plasma was fed into the analysis means. The hydrogen ion concentration of the plasma was analyzed in the analysis means to obtain the value of pH 7.4, which was the same value as in the above-described case, and was substantially equal to a value of the healthy person's plasma.

### Comparative Example

With conventional blood analysis apparatus shown in FIG. 3, whole blood was drawn in, and separated into plasma and blood cell fractions by centrifugation, and plasma fraction was drawn into analysis means. A U-shaped flow channel of the used apparatus had a width of 500 µm, and a depth of 100 µm. Also in this case, one analysis means was disposed, and an electrode formed of carbon paste, coated with a hydrogen ion sensitive film, was used. 1 µL of human whole blood was introduced the blood analysis apparatus through a blood inlet port 204. An electromagnetic pump attached to a outlet port 205 was used in introducing the blood, and a negative suction pressure at this time was -7 kPa from the atmospheric pressure.

After introducing the blood into the blood analysis apparatus as shown in FIG. 3(C), the blood analysis apparatus was set in a centrifugal separator shown in FIG. 7, and centrifugation was performed (10,000 rpm, 2,250 G for 1 min). By centrifugation, plasma fractions 309, 310 and blood cell component 311 were separated in a single U-shaped tube as shown in FIG. 3(D). Different from the result of a blood analysis apparatus of FIG. 5 which has a blood cell reservoir midway in a flow channel, the plasma fractions 309, 310 on upstream and downstream sides of a single U-shaped flow channel were insulated by the blood cell fraction 311.

Thereafter, an electromagnetic pump was connected to draw-in ports 304, 305 position on downstream of the analysis means, and the plasma fraction 310 on the downstream side of the single U-shaped flow channel was fed into analysis means 306, 307. Even when the negative suction pressure at this time was set to -7 kPa equal to that of the first example, it was not possible to draw in the plasma. When the negative suction pressure was gradually raised, and reached -38 kPa from the atmospheric pressure, the plasma 310 on the downstream side of the flow channel started moving. With this movement, the blood cell component in the lower part of the U-shaped flow channel and the upstream-side plasma component simultaneously moved. As compared with the plasma draw-in pressure of the first example, a larger plasma draw-in pressure was required in the comparative example. Accordingly, an effect of the present invention became clearer.

Such an excessively large draw-in pressure is required, and this is supposedly because the plasma fraction has to be drawn in together with the blood cell fraction stuck to the wall of the flow channel. Furthermore, in the single U-shaped blood analysis apparatus shown in FIG. 3, even when the plasma is drawn in with an excessively large draw-in pressure, the plasma actually drawn into the analysis means is an only plasma fraction separated on the downstream side, and the plasma on the upstream side closed by the blood cell fraction cannot be drawn into the analysis means. In the conventional blood analysis apparatus, only about half of the separated plasma cannot be fed into the analysis means in the conventional blood analysis apparatus. On the other hand, in the present blood analysis apparatus having a blood cell reservoir as shown in FIG. 5, all separated plasma can be fed into the analysis means. Therefore, when the equal amount of plasma is fed into the analysis means, there is an advantage that an amount of blood to be introduced into the blood analysis apparatus may be about half only in the present invention as compared with a conventional case.

### Second Example

A blood analysis apparatus 220 having a blood cell reservoir 501A was prepared as shown in FIG. 9(A). Blood was introduced into the blood analysis apparatus to perform centrifugation, and thereafter a plasma fraction 602 was drawn in. It is to be noted that dimensions of the blood analysis apparatus and a flow channel design in this Example are substantially similar to those shown in FIG. 8. As shown in FIG. (9B), this blood analysis apparatus is different from that of FIG. 8 in that an area of an interface between the separated plasma 602 and a blood cell fraction 603 is reduced as compared with a blood analysis apparatus shown in FIG. 8, when the blood is introduced onto the blood analysis apparatus to perform the centrifugation. Accordingly, a draw-in force applied to the blood cell fraction when sucking the plasma is reduced, the surface of the blood cell fraction can be prevented from being disturbed and mixed with the plasma, and substantially all plasma is securely moved into analysis means.

When 1 µL of human whole blood was actually introduced from the blood inlet port to perform the centrifugation, the blood cell and plasma fractions were separated as shown in FIG. 9(B). Thereafter, a pump was connected to a draw-in port 801, and the plasma fraction 602 only could be drawn in the analysis means 802 at a pressure of -7 kPa from the atmospheric pressure. At this time, a blood cell component in a blood cell reservoir does not move, and remains as such in the blood cell reservoir.

### Third Example

As shown in FIG. 10(A), a flow channel width of a lower part of a U-shaped flow channel 303 was increased to constitute a blood cell reservoir 501B, and accordingly a blood analysis apparatus 230 was prepared. In the same manner as in the first and second examples, attempts were made to introduce blood, separate plasma from the blood cell fraction, and draw in plasma fraction. By centrifugation after the blood introduction, as shown in FIG. 10(B), plasma 602 and blood cell fraction 603 were separated, and the plasma fraction 602 only was drawn in without disturbing the blood cell fraction 603, and could be fed into analysis means 802. From the present example, it is seen that it is especially important that the plasma fraction should continuously exist without being divided by the blood cell fraction, when the blood is separated into the plasma and blood cell fractions by centrifugation. This also applies to the blood analysis apparatus having the blood cell reservoir midway in the flow channel according to the first and second examples.

### Fourth Example

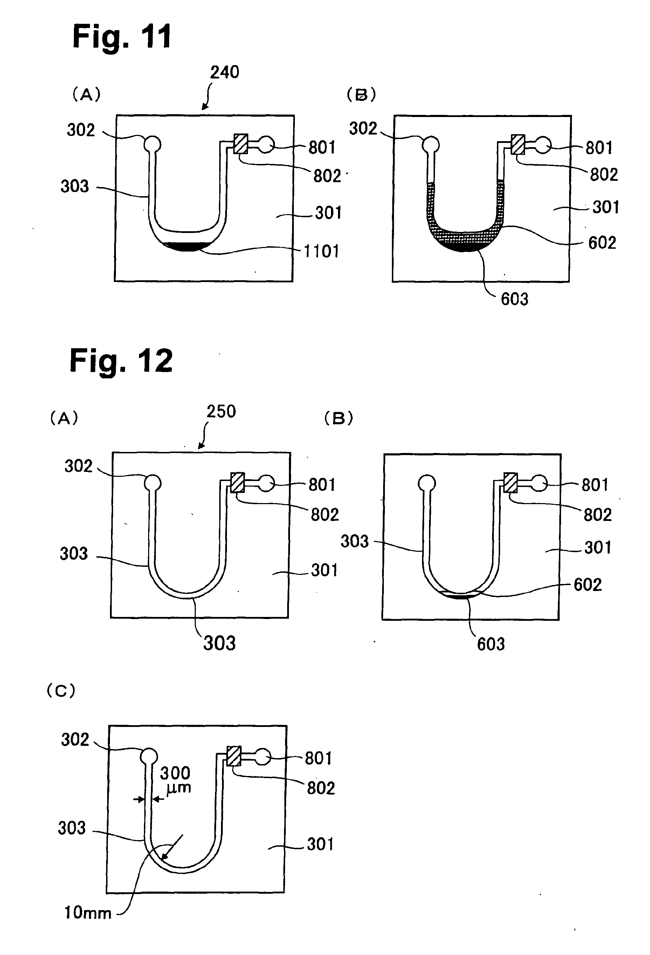

A blood analysis apparatus was prepared in which a flow channel width and flow channel depth of a lower part of a U-shaped flow channel 303 were increased. More specifically, a blood analysis apparatus 240 was prepared in which the depth of a slant line portion 1101 of the lower part of the U-shaped flow channel 303 shown in FIG. 11(A) was set to 300 µm larger than a depth of other flow channel portions which was 100 µm deep. In the same manner as in the third example, introduction of blood, separation of plasma, and draw-in of plasma fraction were tried. The blood was separated into plasma 602 and blood cell fraction 603 by centrifugation as shown in FIG. 11(B), and the plasma fraction 602 only was drawn in without moving the blood cell fraction 603, and could be fed into analysis means 802.

It is to be noted here that depth of the lower part 1101 of the U-shaped flow channel of FIG. 11(A) is large, a volume of this portion is three times as large as that of a blood analysis apparatus of FIG. 10, and therefore blood cell components are accumulated here. Therefore, when blood is introduced into the blood analysis apparatus with an amount equal to that of the third example to perform centrifugation, a ratio of a constituting area of the blood cell fraction 603 seen from a top surface of FIG. 11 with respect to an area occupied by the plasma fraction is smaller than that of FIG. 10. Accordingly, since the width constituted by the plasma fraction in the lower part of the U-shaped flow channel is larger than that of FIG. 10(B), the plasma fraction can be drawn in at a smaller draw-in pressure, when drawing in the plasma fraction after the separation of the blood cell and plasma.

### Fifth Example

A single U-shaped flow channel blood analysis apparatus was prepared as shown in FIG. 12(A), and introduction of blood, separation of plasma/blood cell fractions, and draw-in of plasma fraction were tried in the same manner as in the above-described examples. At this time, a width or depth of a flow channel was determined from an amount of introduced blood (which corresponds to the amount of required plasma). More specifically, the plasma fraction was constituted to continuously exist as shown in FIG. 12(B) without being divided by a blood cell fraction after centrifugation. In actual, a blood analysis apparatus 250 having a single U-shaped flow channel 303 having a flow channel depth of 100 µm was prepared with a dimension shown in FIG. 12(C), 0.1 µL of blood was introduced onto the blood analysis apparatus, and the centrifugal separation was performed. As shown in FIG. 12(B), the blood was separated into a plasma fraction 602 and a blood cell fraction 603, and thereafter all separated plasma fraction only could be fed into analysis means 802. Thus, in the case that an amount of blood to be introduced is estimated based on a plasma amount required and a flow channel is designed so as to prevent the plasma fraction from being divided by the blood cell fraction in the flow channel after centrifugation, a blood cell reservoir shown in FIG. 5 is not necessarily required. The lower most portion of the U-shaped flow channel according to the specific flow channel design can serve as a blood cell reservoir (blood cell fraction container).

### Industrial Applicability

As described above, in the present blood analysis apparatus, a blood cell fraction container for collecting blood cell fraction is provided in a part of a flow channel, which introduces blood into the apparatus, to be positioned in a centrifugal force pressurizing direction, so that the upstream side of the flow channel from the container communicates with that on the downstream side. Accordingly, plasma fractions in the upstream and downstream portions of the flow channel can continuously exist without being divided by the blood cell fraction after the centrifugation. Therefore, as compared with a conventional flow channel constitution, the plasma in a required amount can be fed into the analysis means with a smaller amount of the whole blood, simply a half amount of a blood sample as compared with the prior apparatus. The whole blood can be utilized more efficiently, which is suitable for the shortening the flow channel and the reduction of the apparatus size. Furthermore, the amount of the blood to be collected is decreased, thereby reducing a burden on a subject.

Moreover, the separated plasma fraction, which is not divided by the blood cell fraction, can be transferred due to a lower negative suction pressure. Thus, the pump capacity required in drawing in the plasma can be reduced, which contributes to the size reduction and cost down of peripheral devices.

## Claims

1. A blood analysis apparatus comprising: a flow channel which connects between a blood inlet port and an outlet port; and plasma separating means disposed midway in the flow channel,
wherein said flow channel has an upstream portion of the flow channel elongated along a centrifugal force pressurizing direction and a downstream portion elongated in a direction opposite to the a centrifugal force pressurizing reverse direction;
wherein said plasma separating means is positioned between the upstream and downstream portions of the flow channel and includes a blood cell fraction container which is located in the centrifugal force pressurizing direction side, and in which a blood cell fraction is precipitated and
received; and
wherein the upstream and downstream portions of said flow channel are brought into contact with the blood cell fraction container, and are constituted to communicate with each other in an upper space of the blood cell fraction container.

2. The blood analysis apparatus according to claim 1, wherein at least a part of said flow channel is formed as a U-shaped flow channel, and a lowermost portion of the U-shaped flow channel constitutes said blood cell fraction container.

3. The blood analysis apparatus according to claim 1, wherein at least a part of said flow channel is formed as a U-shaped flow channel, and a lowermost portion of the U-shaped flow channel constitutes said blood cell fraction container, and
a capacity of the blood cell fraction container positioned in a centrifugal force pressurizing direction from an upper inner wall of the lowermost portion of the U-shaped flow channel is larger than the amount of the blood cell fraction in the blood introduced into the flow channel.

4. The blood analysis apparatus according to claim 1, further comprising:
analysis means for analyzing components of the plasma, the analysis means being disposed between said plasma separating means and said outlet port.

5. The blood analysis apparatus according to claim 1, wherein a blood collecting needle is attachable to said blood inlet port.

6. A plasma separation method comprising the following steps of:
(1) providing a chip-shaped blood analysis apparatus comprising:
a flow channel for connecting a blood inlet port to an outlet port, the flow channel having an upstream portion elongated along a centrifugal force pressurizing direction from the blood inlet port and a downstream portion elongated in a direction opposite to the centrifugal force pressurizing direction; and
plasma separating means located between the upstream and downstream portions of the flow channel and including a blood cell fraction container which is located in a centrifugal force pressurizing direction side and in which the blood cell fraction is precipitated and received, the upstream and downstream portions of the flow channel being brought into contact with the blood cell fraction container and constituted to communicate with each other in an upper space of the blood cell fraction container;
(2) introducing a whole blood sample into the flow channel from the blood inlet port; and
(3) centrifuging the blood analysis apparatus in such a manner that the blood cell fraction container is disposed in the centrifugal force pressurizing direction so as to precipitate blood cell components in a blood sample in said blood cell fraction container, so that the plasma separated as centrifugal supernatant is allowed to continuously exist in both of the upstream and downstream portions of the flow channel while the plasma contacts with blood cell fraction in the blood cell fraction container.

7. The plasma separation method according to claim 6, wherein said blood analysis apparatus further comprises analysis means for analyzing the components in the plasma, the analysis mean being disposed between the plasma separating means and the outlet port, and
wherein the plasma continuously existing in the upstream and downstream portions of said flow channel is fed into the analysis means after said step (3).
